# EUROPÄISCHE PATENTSCHRIFT

(11) **EP 1 288 192 B1**
(45) Veröffentlichungstag und Bekanntmachung des Hinweises auf die Patenterteilung: **18.07.2007**
(21) Anmeldenummer: 02017673.1
(22) Anmeldetag: 07.08.2002
(51) Int. Cl.: C07C 227/06, C07C 229/50, C09B 1/514

(54) **Verfahren zur Herstellung von 1-Amino-4-hydroxyanthrachinonen**
Process for preparing 1-amino-4-hydroxyanthraquinones
Procédé de préparation de 1-amino-4-hydroxyanthraquinones

(30) Priorität: 16.08.2001 DE 10140107
(43) Veröffentlichungstag der Anmeldung: 05.03.2003
(73) Patentinhaber: LANXESS Deutschland GmbH, 51369 Leverkusen (DE)
(72) Erfinder: Thiebes, Christoph, Dr., 50670 Köln (DE); Stawitz, Josef-Walter, Dr., 51519 Odenthal (DE)

(56) Entgegenhaltungen:
- CH-A- 391 930
- CH-A4- 1 337 167
- DE-A- 2 041 374
- US-A- 1 931 264
- US-A- 2 419 405

## Beschreibung

Die Erfindung betrifft ein Verfahren zur Herstellung von 1-Amino-4-hydroxyanthrachinonen.

1-Amino-4-hydroxyanthrachinone sind beispielsweise als Farbstoffe für Kunststoffe und Synthesefasern als auch für Vorprodukte zur Herstellung von Wollfarbstoffen bekannt. Bislang wurden diese Verbindungen hergestellt, indem man 1,4-Dihydroxyanthrachinon (Chinizarin) gegebenenfalls im Gemisch mit 2,3-Dihydro-1,4-dihydroxyanthrachinon (Leukochinizarin) mit Aminen umsetzt, wobei die Reaktion gegebenenfalls in Gegenwart von Kondensationshilfsmitteln durchgeführt wurde.

So gelingt beispielsweise gemäß US-A-193 12 64 die Unterdrückung der Bis-Addukt-Bildung durch ein wässriges Lösungsmittel, jedoch sind gleichzeitig Aminüberschüsse nötig, um gute Ausbeuten zu erhalten. In GB-A 20 13 701 erfolgt die Umsetzung in Gegenwart von oberflächenaktiven Substanzen. DE-A-161 96 46 und US-A-2 419 405 beschreibt die Verwendung von Alkoholen als Lösungsmittel.

Nachteilig bei allen Verfahrensweisen ist die niedrige Raum-Zeit-Ausbeute und die Tatsache, dass der erhaltene Farbstoff nicht brillant genug ist.

Aufgabe der vorliegenden Erfindung ist die Bereitstellung eines Verfahrens, welches die Nachteile des Standes der Technik nicht mehr besitzt.

Es wurde nun ein Verfahren zur Herstellung von 1-Amino-4-hydroxyanthrachinonen der Formel (I) 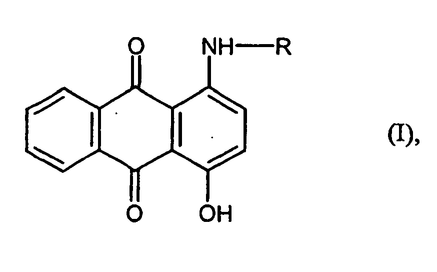 worin
- R: ein aliphatischer oder aromatischer Rest ist,
gefunden, das dadurch gekennzeichnet ist, dass man 1,4-Dihydroxyanthrachinone mit aliphatischen oder aromatischen Aminen in Gegenwart von N-Methyl-2-pyrrolidon umsetzt.

Die bevorzugten, bei dem erfindungsgemäßen Verfahren zum Einsatz kommenden aliphatischen oder aromatischen Amine sind primär. Die aliphatischen Amine können beispielsweise gesättigt, ungesättigt, verzweigt oder geradkettig sein. Besonders bevorzugte aliphatische Amine sind beispielsweise solche der nachstehenden Formeln:

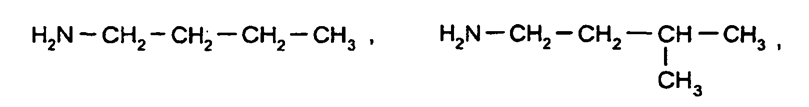

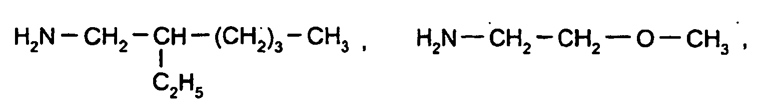

H₂N-CH₂-CH₂-CH₂-O-CH₃, H₂N-(CH₂)₃-O-C₂H₅,

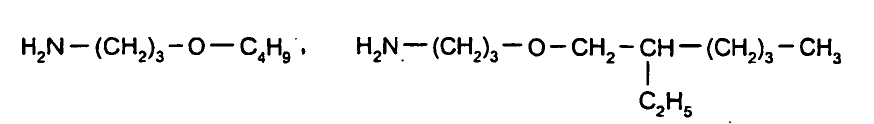

und H₂N-(CH₂)₃-O―C₁₈H₃₇

Besonders vorteilhaft wird das erfindungsgemäße Verfahren jedoch zur Herstellung von 1-Arylamino-4-hydroxyanthrachinonen verwendet, bei denen die aromatischen Amine primär sind, und insbesondere der Formel (II) 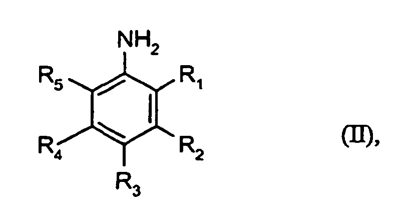 entsprechen, worin
- R₁, R₂, R₃, R₄ und R₅: unabhängig voneinander für H, C₁-C₁₂-Alkyl, insbesondere C₁-C₄-Alkyl, Halogen, C₁-C₄-Alkoxy oder C₆-C₁₀-Aryloxy bedeuten und
- R₂: auch für -SO₂-NH-R₆ stehen kann, wobei R₆ für gegebenenfalls substituiertes Aryl, insbesondere C₆-C₁₀-Aryl, wie Phenyl oder Naphthyl, oder Alkyl, insbesondere C₁-C₄-Alkyl, wie Methyl, Ethyl, Propyl oder Butyl, steht und als mögliche Substituenten C₁-C₄-Alkyl, OH, Halogen, C₁-C₄-Alkoxy auch C₉-C ₁₀-Aryloxy bevorzugt sind.

### Besonders bevorzugt sind die folgenden aromatische Amine der Formel (II):

Anilin, o-Toluidin, 3,5-Dimethylanilin, 2,4-Dimethylanilin, p-Toluidin sowie p-Aminoacetanilid.

Das im erfindungsgemäßen Verfahren eingesetzte 1,4-Dihydroxyanthrachinon (Chinizarin) wird bevorzugt im Gemisch mit seiner Leukoform dem 2,3-Dihydro-1,4-dihydroxyanthrachinon (Leukochinizarin) eingesetzt, wobei die Leuko-Verbindung vorzugsweise in einer Menge von 1 bis 90 Gew.-%, vorzugsweise 1 bis 20 Gew.-%, besonders bevorzugt 3 bis 10 Gew.-%, bezogen auf die Summe von Chinizarin und Leukochinizarin eingesetzt wird. Das Gemisch aus Leukochinizarin und Chinizarin kann beispielsweise aus dem Chinizarin durch Zugabe von Reduktionsmitteln wie Zinkstaub oder Natriumdithionit in situ gebildet werden. Die Anthrachinonverbindungen Chinizarin und ihre Leukoform können aber auch separat hergestellt werden.

Das Verhältnis von Amin zu Anthrachinonverbindung, d.h. Gesamtmenge aus Chinizarin und Leukochinizarin, wird vorzugsweise so gewählt, daß 1 bis 2 mol-Äquivalente, besonders bevorzugt 1,1 bis 1,3 mol-Äquivalente Amin pro mol des Anthrachinons (Gesamtmenge an Chinizarin und Leukochinizarin) entfallen.

In einer besonders bevorzugten Ausführungsform wird das erfindungsgemäße Verfahren in Gegenwart von Borsäure durchgeführt. Vorzugsweise wird diese in einer Menge von 0,025 bis 1 mol-Äquivalent, bezogen auf die Menge des Anthrachinons (Gesamtmenge an Chinizarin und Leukochinizarin), insbesondere 0,025 bis 0,4 mol-Äquivalente, eingesetzt.

Das Verfahren kann gegebenenfalls in Gegenwart von weiteren, insbesondere mit NMP mischbaren, organischen Lösungsmitteln durchgeführt werden. Geeignete Lösungsmittel sind beispielsweise aliphatische Alkohole, wie n-Butanol oder isoAmylalkohol oder Wasser. Auch im Überschuß eingesetztes Amin kann als organisches Lösungsmittel dienen. Die Menge an weiteren Lösungsmitteln, insbesondere an Wasser, kann beispielsweise 1 bis 30 Gew.-% an der Reaktionsmischung ausmachen, bevorzugt wird ohne Zusatz eines weiteren Lösungsmittels gearbeitet.

Die Menge an NMP, bezogen auf die Gesamtmenge der Reaktionsmischung beträgt vorzugsweise 30 bis 75 Gew.-%, insbesondere 40 bis 55 Gew.-%.

Das erfindungsgemäße Verfahren wird vorzugsweise bei einer Temperatur von 60 bis 160°C, vorzugsweise bei 70 bis 130°C, insbesondere bei 85 bis 105°C, durchgeführt.

Der Reaktionsmischung kann Wasser zugesetzt werden, außerdem entsteht durch die Reaktion Wasser. Wasser kann während der Reaktion teilweise entfernt werden, beispielsweise durch Destillation. Vorzugsweise verfährt man in einer Weise, dass die Reaktionsschmelze nach beendeter Reaktion 1 bis 10, insbesondere 2 bis 3 Gew.-% Wasser enthält.

In einer bevorzugten Ausführungsform des erfindungsgemäßen Verfahrens werden Borsäure und eine Hilfssäure eingesetzt. Als Hilfssäuren werden beispielsweise anorganische oder organische Säuren, insbesondere Hydroxycarbonsäuren eingesetzt.

Die bevorzugt einzusetzenden Hydroxycarbonsäuren sind vorzugsweise aliphatisch oder aromatisch. In einer besonderen Ausführungsform des erfindungsgemäßen Verfahrens tragen die aliphatischen Hydroxycarbonsäuren die Hydroxy- und Carbonsäuregruppe am selben C-Atom. Die aromatischen Hydroxycarbonsäuren tragen die Hydroxy- und die Carbonsäuregruppe vorzugsweise an zwei unmittelbar benachbarten aromatischen C-Atomen.

Als aliphatische Hydroxycarbonsäuren sind besonders die mit 2 bis 7 C-Atomen bevorzugt. Beispielsweise können genannt werden: Hydroxyessigsäure, Milchsäure, Äpfelsäure, Weinsäure, Zitronensäure, 2,2-Bis-(hydroxymethyl)-propionsäure und Galactonsäure. Besonders bevorzugt sind Hydroxyessigsäure sowie Milchsäure.

Als aromatische Hydroxycarbonsäuren sind insbesondere ortho-Hydroxycarbonsäuren des Benzols oder Naphthalins von Bedeutung. Bevorzugt sind Salicylsäuren und ihre Derivate, beispielsweise aliphatische, wie C₁-C₄-Alkyl- oder aromatische, wie C₆-C₁₀-Aryl-Ester, der Formel 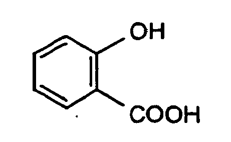 sowie Naphthalin-ortho-hydroxycarbonsäuren und ihre Derivate, beispielsweise aliphatische, wie C₁-C₄-Alkyl- oder aromatische, wie C₆-C₁₀-Aryl-Ester, der Formel 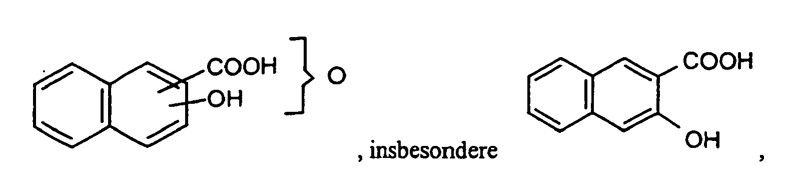 die gegebenenfalls durch ein oder mehrere gleiche oder verschiedene Reste R substituiert sind,
wobei
- R: für H, C₁-C₄-Alkyl, insbesondere CH₃, Halogen, insbesondere Cl, Br und F, OH, CN, COOH oder NO₂ steht.

Beispielsweise können genannt werden: 2,5-Dihydroxy-1,4-benzoldicarbonsäure, 2-Naphthol-3 -carbonsäure.

Das erfindungsgemäße Verfahren kann dabei in Gegenwart von einer oder mehreren Hydroxycarbonsäuren durchgeführt werden.

Die Reaktionsmischung wird nach beendeter Reaktion vorzugsweise abgekühlt. Zur Oxidation von gegebenenfalls vorhandenen Leukoverbindungen kann Luft durch die Reaktionsmischung geleitet werden. Die Oxidation kann jedoch auch mit anderen Oxidationsmittel als Sauerstoff durchgeführt werden. Vorzugsweise wird ohne Oxidation gearbeitet. Danach folgt in der Regel die Isolierung der Anthrachinonverbindung der Formel (I), wobei im allgemeinen mit aliphatischen Alkoholen wie Methanol, Ethanol, Propanol, Butanol oder mit Wasser oder Alkoholgemischen die Anthrachinonverbindung der Formel (I) gefällt wird. Die Anthrachinonverbindung wird filtriert und vorzugsweise mit den genannten Alkoholen gewaschen. In der Regel schließen sich daran noch Wasserwäschen und schließlich die Trocknung an. Ein Vorteil des erfindungsgemäßen Verfahrens ist der, dass die gewünschten Produkte auch ohne Fällung mit einem aliphatischen Alkohol und/oder Wasser in ausgezeichneten Ausbeuten und Qualitäten erhalten werden.

Das erfindungsgemäße Verfahren zeichnet sich durch eine exzellente Raum-Zeit-Ausbeute und zum anderen durch verbesserte Verfahrensprodukte aus.

Die nach dem erfindungsgemäßen Verfahren hergestellten Farbstoffe eignen sich besonders zum Massefärben von Kunststoffen.

Unter Massefärben werden hierbei insbesondere Verfahren verstanden, bei denen der Farbstoff in die geschmolzene Kunststoffinasse eingearbeitet wird, z.B. unter Zuhilfenahme eines Extruders, oder bei denen der Farbstoff bereits Ausgangskomponenten zur Herstellung des Kunststoffes, z.B. Monomeren vor der Polymerisation, zugesetzt wird.

Besonders bevorzugte Kunststoffe sind Thermoplaste, beispielsweise Vinylpolymere, Polyester, Polyamide sowie Polyolefine, insbesondere Polyethylen und Polypropylen oder Polycarbonate.

Geeignete Vinylpolymere sind Polystyrol, Styrol-Acrylnitril-Copolymere, Styrol-Butadien-Copolymere, Styrol-Butadien-Acrylnitril-Terpolymere, Polymethacrylat, Polyvinylchlorid u.a.

Weiterhin geeignet sind Polyester wie beispielsweise Polyethylenterephthalate, Polycarbonate und Celluloseester.

Bevorzugt sind Polystyrol, Styrol-Mischpolymere, Polycarbonate, Polymethacrylate und Polyamide. Besonders bevorzugt ist Polystyrol, Polyethylen und Polypropylen.

Die erwähnten hochmolekularen Verbindungen können einzeln oder in Gemischen, als plastische Massen oder Schmelzen vorliegen.

Die nach dem erfindungsgemäßen Verfahren erhaltenen Farbstoffe werden vorzugsweise in feinverteilter Form zur Anwendung gebracht, wobei Dispergiermittel mitverwendet werden können aber nicht müssen.

Wird die Farbstoffinischung nach der Polymerisation eingesetzt, so werden sie vorzugsweise mit dem Kunststoffgranulat trocken vermischt oder vermahlen und dieses Gemisch z.B. auf Mischwalzen oder in Schnecken plastifiziert und homogenisiert. Man kann die Farbstoffe aber auch der schmelzflüssigen Masse zugeben und diese durch Rühren homogen verteilen. Das derart vorgefärbte Material wird dann wie üblich z.B. durch Verspinnen zu Borsten, Fäden usw. oder durch Extrusion oder im Spritzguss-Verfahren zu Formteilen weiterverarbeitet.

Da die Farbstoffe gegenüber Polymerisationskatalysatoren insbesondere Peroxiden, beständig sind, ist es auch möglich, den Farbstoff den monomeren Ausgangsmaterialien für die Kunststoffe zuzusetzen und dann in Gegenwart von Polymerisationskatalysatoren zu polymerisieren. Dazu wird der Farbstoff vorzugsweise in den monomeren Komponenten gelöst oder mit ihnen innig vermischt.

Die nach dem erfindungsgemäßen Verfahren erhaltenen Farbstoffe werden vorzugsweise zum Färben der genannten Polymeren in Mengen von 0,0001 bis 1 Gew.-%, insbesondere 0,01 bis 0,5 Gew.-%, bezogen auf die Polymermenge, eingesetzt.

Durch Zusatz von in den Polymeren unlöslichen Pigmenten, wie z.B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-%, bezogen auf die Polymermenge, verwendet werden.

Nach dem erfindungsgemäßen Färbe-Verfahren erhält man transparente bzw. gedeckte brillante violette Färbungen mit guter Hitzebeständigkeit sowie guter Licht-, Wetter- und Sublimierechtheit.

In das erfindungsgemäße Färbe-Verfahren können auch Mischungen der Farbstoffe der Formel (I) mit anderen Farbstoffen und/oder anorganischen bzw. organischen Pigmenten eingesetzt werden.

Die Erfindung wird erläutert durch die folgenden Beispiele, in denen die Teile gewichtsmäßig angegeben sind, Prozentangaben Gewichtsprozente (Gew.-%) bedeuten.

### Beispiele

### Beispiel 1

### 1 -p-Toluidino-4-hydroxyanthrachinon:

In einem 1 000 ml 4-Halskolben werden unter kräftigem Rühren 271 Teile (1,13 mol) Chinizarin, 17 Teile (0,07 mol) Dihydrochinizarin, 157 Teile (1,47 mol) p-Toluidin, 4 Teile (0,065 mol) Borsäure, 4,5 Teile Wasser und 35,5 Teile (0,355 mol) 90 % Milchsäure in 413 Teile N-Methylpyrrolidon eingetragen. Man erhitzt auf 90°C und hält diese Temperatur für 12h. Anschließend kühlt man ab und filtriert. Man wäscht das erhaltene Produkt mit 500 Teilen 60°C heißem Methanol und trocknet. Man erhält 370 Teile (94 % Ausbeute) eines dunkelvioletten Produktes, welches bei der Einfärbung von Polystyrol ein sehr klares Violett liefert.

### Beispiel 2

### 1-Anilino-4-hydroxyanthrachinon:

Man verfährt wie in Beispiel 1, nur dass man 157 Teile p-Toluidin durch 136 Teile (1,46 mol) Anilin ersetzt. Man erhält 312 Teile (83 % Ausbeute) eines dunkelvioletten Produktes, welches bei der Einfärbung von Polystyrol einen violetten Farbton liefert. Der Farbton ist bei vergleichbarer Farbstärke deutlich röter als der des in Beispiel 1 beschriebenen Farbstoffes.

### Beispiel 3

### 1-p-Acetamidoanilino-4-hydroxyanthrachinon:

Man verfährt wie in Beispiel 1, nur dass man 157 Teile p-Toluidin durch 220 Teile (1,47 mol) p-Aminoacetanilid ersetzt und statt 400 Teilen 600 Teile NMP verwendet. Man erhält 312 Teile eines dunkelvioletten Produktes, welches bei der Einfärbung von Polystyrol ein sehr klares Violett liefert. Der Farbton ist bei vergleichbarer Farbstärke etwas blaustichiger als der des in Beispiel 1 beschriebenen Farbstoffes.

### Beispiel 4

### 1-(3',5'-Dimethylanilino)-4-hydroxyanthrachinon:

Man verfährt wie in Beispiel 1, nur dass man 157 Teile p-Toluidin durch 178 Teile (1,47 mol) 3,5-Dimethylanilin ersetzt. Man erhält 336 Teile eines dunkelvioletten Produktes, welches bei der Einfärbung von Polystyrol ein sehr klares Violett liefert. Der Farbton ist bei vergleichbarer Farbstärke etwas rotstichiger und klarer als der des in Beispiel 1 beschriebenen Farbstoffes.

## Patentansprüche

1. Verfahren zur Herstellung von 1-Amino-4-hydroxyanthrachinonen der Formel (I), 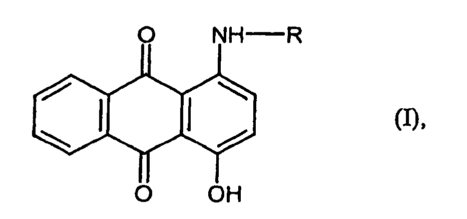 worin
R ein aliphatischer oder aromatischer Rest ist,
**dadurch gekennzeichnet, dass** man 1,4-Dihydroxyanthrachinone mit aliphatischen oder aromatischen Aminen in Gegenwart von N-Methyl-2-pyrrolidon umsetzt.

2. Verfahren gemäß Anspruch 1, **dadurch gekennzeichnet, dass** die Umsetzung mit einem aromatischen Amin erfolgt.

3. Verfahren gemäß Anspruch 1, **dadurch gekennzeichnet, dass** die Umsetzung mit einem aromatischen Amin der Formel (II) 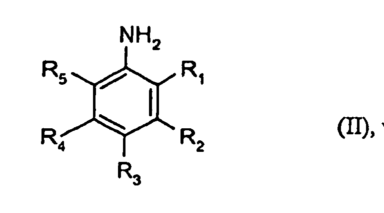 worin
R₁, R₂, R₃, R₄ und R₅ unabhängig voneinander für H, C₁-C₁₂-Alkyl, insbesondere C₁-C₄-Alkyl, Halogen, C₁-C₄-Alkoxy oder C₆-C₁₀-Aryloxy bedeuten und
R₂ auch für -SO₂-NH-R₆ stehen kann, wobei R₆ für gegebenenfalls substituiertes Aryl, insbesondere C₆-C₁₀-Aryl, wie Phenyl oder Naphthyl, oder Alkyl, insbesondere C₁-C₄-Alkyl, wie Methyl, Ethyl, Propyl oder Butyl, steht und als mögliche Substituenten C₁-C₄-Alkyl, OH, Halogen, C₁-C₄-Alkoxy auch C₉-C₁₀-Aryloxy bevorzugt sind,
erfolgt.

4. Verfahren gemäß Anspruch 1, **dadurch gekennzeichnet, dass** als aromatisches Amin Anilin, o-Toluidin, 3,5-Dimethylanilin, 2,4-Dimethylanilin, p-Toluidin oder p-Aminoacetanilid eingesetzt wird.

5. Verfahren gemäß Anspruch 1, **dadurch gekennzeichnet, dass** die Umsetzung in Gegenwart von Borsäure erfolgt.

6. Verfahren gemäß Anspruch 1, **dadurch gekennzeichnet, dass** die Umsetzung in Gegenwart einer Hilfssäure, insbesondere einer Hydroxycarbonsäure erfolgt.

## Claims

1. Process for preparing 1-amino-4-hydroxyanthraquinones of the formula (I), 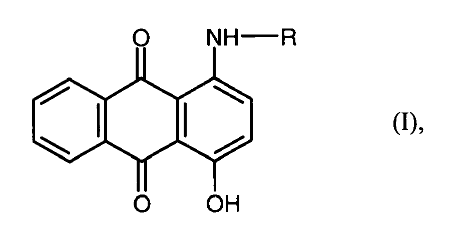 where
R is an aliphatic or aromatic radical,
**characterized in that** 1,4-dihydroxyanthraquinones are reacted with aliphatic or aromatic amines in the presence of N-methyl-2-pyrrolidone.

2. Process according to Claim 1, **characterized in that** the reaction is carried out with an aromatic amine.

3. Process according to Claim 1, **characterized in that** the reaction is carried out with an aromatic amine of the formula (II) 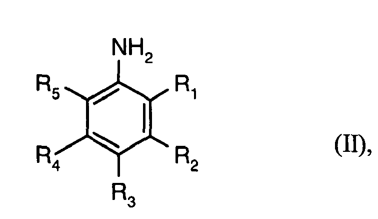 where
R₁, R₂, R₃, R₄ and R₅ are singly H, C₁-C₁₂-alkyl, especially C₁-C₄-alkyl, halogen, C₁-C₄-alkoxy or C₆-C₁₀-aryloxy, and
R₂ may additionally be -SO₂-NH-R₆, where R₆ is optionally substituted aryl, especially C₆-C₁₀-aryl, such as phenyl or naphthyl, or alkyl, especially C₁-C₄-alkyl, such as methyl, ethyl, propyl or butyl, possible substituents preferably being selected from C₁-C₄-alkyl, OH, halogen, C₁-C₄-alkoxy and C₉-C₁₀-aryloxy.

4. Process according to Claim 1, **characterized in that** the aromatic amine used is aniline, o-toluidine, 3,5-dimethylaniline, 2,4-dimethylaniline, p-toluidine or p-aminoacetanilide.

5. Process according to Claim 1, **characterized in that** the reaction is carried out in the presence of boric acid.

6. Process according to Claim 1, **characterized in that** the reaction is carried out in the presence of an auxiliary acid, especially a hydroxy carboxylic acid.

## Revendications

1. Procédé de préparation de 1-amino-4-hydroxy-anthraquinones de formule (I), 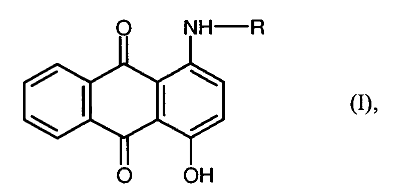 dans laquelle :
R est un radical aliphatique ou aromatique,
**caractérisé en ce que** des 1,4-dihydroxyanthraquinones sont mises à réagir avec des amines aliphatiques ou aromatiques en présence de N-méthyl-2-pyrrolidone.

2. Procédé selon la revendication 1, **caractérisé en ce que** la réaction est effectuée avec une amine aromatique.

3. Procédé selon la revendication 1, **caractérisé en ce que** la réaction est effectuée avec une amine aromatique de formule (II) 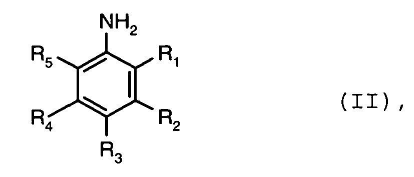 dans laquelle
R₁, R₂, R₃, R₄ et R₅ représentent, indépendamment les uns des autres, un atome d'hydrogène, un groupe alkyle en C₁₋₁₂, en particulier un groupe alkyle en C₁₋₄, un atome d'halogène, un groupe alcoxy en C₁₋₄ ou un groupe aryloxy en C₆₋₁₀, et
R₂ peut également représenter un groupe -SO₂-NH-R₆, dans lequel R₆ représente un groupe aryle éventuellement substitué, en particulier un groupe aryle en C₆₋₁₀, tel qu'un groupe phényle ou un groupe naphtyle, ou un groupe alkyle, en particulier un groupe alkyle en C₁₋₄, tel qu'un groupe méthyle, un groupe éthyle, un groupe propyle ou un groupe butyle, et en tant que substituants possibles, on préfère un groupe alkyle en C₁₋₄, un groupe OH, un atome d'halogène, un groupe alcoxy en C₁₋₄ et un groupe aryloxy en C₉₋₁₀.

4. Procédé selon la revendication 1, **caractérisé en ce que** l'on utilise, en tant qu'amine aromatique, l'aniline, la o-toluidine, la 3,5-diméthylaniline, la 2,4-diméthylaniline, la p-toluidine ou le p-aminoacétanilide.

5. Procédé selon la revendication 1, **caractérisé en ce que** la réaction est effectuée en présence d'acide borique.

6. Procédé selon la revendication 1, **caractérisé en ce que** la réaction est effectuée en présence d'un acide auxiliaire, en particulier d'un acide hydroxycarboxylique.
